# EUROPEAN PATENT SPECIFICATION

(11) **EP 3 962 580 B1**
(45) Date of publication and mention of the grant of the patent: **23.08.2023**
(21) Application number: 20720003.1
(22) Date of filing: 16.04.2020
(51) Int. Cl.: A61N 1/05, A61B 17/22

(54) **LEAD ENGAGEMENT DEVICE**
LEITUNGSEINGRIFFVORRICHTUNG
DISPOSITIF D'ENGAGEMENT DE CONDUCTEUR

(30) Priority: 30.04.2019 US 201962840742 P
(43) Date of publication of application: 09.03.2022
(73) Proprietor: Koninklijke Philips N.V., 5656 AG Eindhoven (NL)
(72) Inventor: BOWE, Wade, Allen, 5656 AE Eindhoven (NL); PAYNE, Jeff, 5656 AE Eindhoven (NL)
(74) Representative: Philips Intellectual Property & Standards
(86) International application number: PCT/EP2020/060677
(87) International publication number: WO 2020/221593

(56) References cited:
- EP-A2- 1 848 497
- US-A1- 2004 236 396
- US-A1- 2018 028 779
- US-A1- 2019 030 324

## Description

### FIELD OF THE DISCLOSURE

The devices described herein generally relate to lead engagement devices for engaging and facilitating removal of an implanted lead, such as a cardiac implantable electronic device ("CIED") lead, from a patient's body, and more specifically relate to lead engagement devices providing relatively large areas for engaging implanted leads.

### BACKGROUND

Various medical procedures attach wire-like devices to internal portions of a patient's body, such as an electrical lead for a cardiac implantable electronic device ("CIED"). CIED leads are electrically conductive wires which run to an electrode that is attached to an inner wall of a patient's heart. CIED leads are typically a coil of wire enclosed in an outer cylindrical sheath of electrically insulating material. The coil of wire usually leaves a hollow space running down the center of the CIED lead (a "lumen").

CIED leads are usually implanted with the intention that they will remain in the patient for several years. During such time, fibrous tissue grows over the electrode and portions of the lead. CIED leads are often provided with additional barb-like structures or a corkscrew type of structure to encourage adhesion to the inner wall of the patient's heart.

CIED leads sometimes need to be removed for a variety of reasons including infection, malfunction, venous occlusion, advisory, etc.

Numerous lead engagement devices have thus been developed that can be inserted into the lumen of a CIED lead and attached to the CIED lead in order to apply traction to the lead. However, these devices typically have a disadvantage that they attach to the CIED lead in a localized area. Applying traction to the CIED lead and/or CIED lead engagement devices can result in the CIED lead becoming distorted and/or breaking before it can be removed from the patient.

Accordingly, it is desirable to provide improved lead engagement devices.
US 2004/0236396 A1 discloses a lead locking device that has a lead insertion member having a proximal end and a distal end and has a lead engaging assembly. The lead insertion member defines a lumen extending along a longitudinal axis between the distal end and the proximal end of the lead engaging assembly. A mandrel disposed in the lumen of the lead engaging assembly extends along substantially the entire length of the lumen and protrudes beyond the most proximal end of the lead insertion member. The mandrel includes a distal portion in slidable contact with at least a portion of the lead engaging assembly. The lead engaging assembly has a first configuration while being inserted into a lumen of a lead and a second configuration while engaging the lead from within the lumen of the lead. The lead engaging member has at least two expansion jaws that, in the first configuration, define a substantially cylindrical body. The expansion jaws translate radially outwardly from the longitudinal axis to engage the lumen of the lead when in the second configuration.

### SUMMARY

The invention is defined by the claims. The present disclosure presents a lead engagement device configured to be positioned in a lead lumen of a lead. The lead engagement device includes a hypotube, and the hypotube includes a wall having an inner surface defining an inner lumen and an outer surface opposite the inner surface. A plurality of apertures extend through the wall from the inner surface to the outer surface. A plurality of lead engagement fingers are coupled to the wall and extend outwardly and proximally from the outer surface. Each of the plurality of lead engagement fingers is disposed adjacent to one of the plurality of apertures. The plurality of lead engagement fingers are configured to permit relative motion between the lead engagement device and the lead when applying a first force to the lead engagement device. The plurality of lead engagement fingers are also configured to engage the lead and inhibit relative motion between the lead engagement device and the lead when applying a second force to the lead engagement device, the second force being applied in an opposite direction than the first force.

The lead engagement device according to the previous paragraph, wherein the inner lumen defines a longitudinal axis of the hypotube, the plurality of apertures are disposed in planes substantially parallel to the longitudinal axis of the hypotube, and the plurality of lead engagement fingers are normally disposed at acute angles relative to the longitudinal axis.

The lead engagement device according to any of the previous paragraphs, wherein a plane in which at least one of the acute angles is disposed substantially bisects the wall of the hypotube.

The lead engagement device according to any of the previous paragraphs, wherein at least one of the acute angles is normally in a range of 5 to 45 degrees.

The lead engagement device according to any of the previous paragraphs, wherein the inner lumen defines a longitudinal axis of the hypotube and circumferential direction extending about the longitudinal axis, wherein the plurality of fingers are curved in the circumferential direction.

The lead engagement device according to any of the previous paragraphs, wherein at least one of the plurality of lead engagement fingers has a distal end coupled to the wall, a proximal free end opposite the distal end, and length between the distal end and the proximal free end in a range of 0.25 mm to 6.35 mm (0.01 to 0.25 inches).

The lead engagement device according to any of the previous paragraphs, wherein the plurality of lead engagement fingers are cantilevered from the wall.

The lead engagement device according to any of the previous paragraphs, wherein the plurality of lead engagement fingers monolithically couple to the wall.

The lead engagement device according to any of the previous paragraphs, wherein the plurality of lead engagement fingers have a first shape, the plurality of apertures have a second shape, the second shape being the same as the first shape.

The lead engagement device according to any of the previous paragraphs, wherein the first shape and the second shape are rectangles.

The lead engagement device according to any of the previous paragraphs, further comprising an outer sheath comprising an outer lumen, the outer lumen removably receiving the hypotube.

The present disclosure also presents a method for manufacturing a lead engagement device. The method includes providing a hypotube including a wall, the wall including an inner surface defining an inner lumen and an outer surface opposite the inner surface. The method further includes cutting the wall to form a plurality of lead engagement fingers. The method includes deforming the plurality of lead engagement fingers such that the plurality of lead engagement fingers normally extend outwardly and proximally from the outer surface.

The method according to the previous paragraph, wherein cutting the wall comprises laser cutting the wall.

The method according to any of the previous paragraphs, wherein deforming further comprises forming a plurality of apertures extending through the wall from the inner surface to the outer surface and disposed adjacent to the plurality of lead engagement fingers.

The method according to any of the previous paragraphs, wherein the inner lumen defines a longitudinal axis of the hypotube, and deforming the plurality of lead engagement fingers causes the plurality of lead engagement fingers to be normally disposed at acute angles relative to the longitudinal axis.

The method according to any of the previous paragraphs, wherein a plane in which at least one of the acute angles is disposed substantially bisects the wall of the hypotube.

The method according to any of the previous paragraphs, wherein at least one of the acute angles is in a range of 5 to 45 degrees.

The method according to any of the previous paragraphs, wherein cutting and deforming provides the plurality of lead engagement fingers with a distal end coupled to the wall, a proximal free end opposite the distal end, and length between the distal end and the proximal free end in a range of 0.25 mm to 6.35 mm (0.01 to 0.25 inches).

The method according to any of the previous paragraphs, wherein cutting provides the plurality of lead engagement fingers with rectangular shapes.

It should be understood that every maximum numerical limitation given throughout this disclosure is deemed to include each and every lower numerical limitation as an alternative, as if such lower numerical limitations were expressly written herein. Every minimum numerical limitation given throughout this disclosure is deemed to include each and every higher numerical limitation as an alternative, as if such higher numerical limitations were expressly written herein. Every numerical range given throughout this disclosure is deemed to include each and every narrower numerical range that falls within such broader numerical range, as if such narrower numerical ranges were all expressly written herein.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure may be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
FIG. 1 is a perspective view of a lead engagement device according to an embodiment of the present disclosure.
FIG. 2A is a detail perspective view of a distal lead engagement portion of the lead engagement device of FIG. 1.
FIG. 2B is a side view of the lead engagement portion of FIG. 2A.
FIG. 2C is a transverse sectional view of the lead engagement portion along line 2C-2C of FIG. 2B.
FIG. 2D is a distal end view of the lead engagement portion of FIG. 2A.
FIG. 3A is a transverse sectional view of the lead engagement portion of FIG. 2A disposed in a lumen of a lead.
FIG. 3B is another transverse sectional view of the lead engagement portion of FIG. 2A disposed in the lumen of the lead.
FIG. 4 is a diagram illustrating a method for manufacturing a lead engagement device according to an embodiment of the present disclosure.
FIG. 5A is a perspective view of a lead engagement device according to another embodiment of the present disclosure in a stowed configuration.
FIG. 5B is a perspective view of the lead engagement device of FIG. 5A in a deployed configuration.
FIG. 6A is a transverse sectional view of a lead engagement portion of the lead engagement device of FIG. 5A disposed in a lumen of a lead and in the stowed configuration.
FIG. 6B is another transverse sectional view of a lead engagement portion of the lead engagement device of FIG. 5A disposed in the lumen of the lead and in the deployed configuration.
FIG. 7A is a perspective view of a lead engagement device according to another embodiment of the present disclosure.
FIG. 7B is a side view of the lead engagement device of FIG. 7A.

It should be understood that the drawings are not necessarily to scale. In certain instances, details that are not necessary for an understanding of the disclosure or that render other details difficult to perceive may have been omitted. It should be understood, of course, that the disclosure is not necessarily limited to the particular embodiments illustrated herein.

### DETAILED DESCRIPTION

Before any embodiments of the disclosure are explained in detail, it is to be understood that the disclosure is not limited in its application to the details of construction and the arrangement of components set forth in the following description or illustrated in the following drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

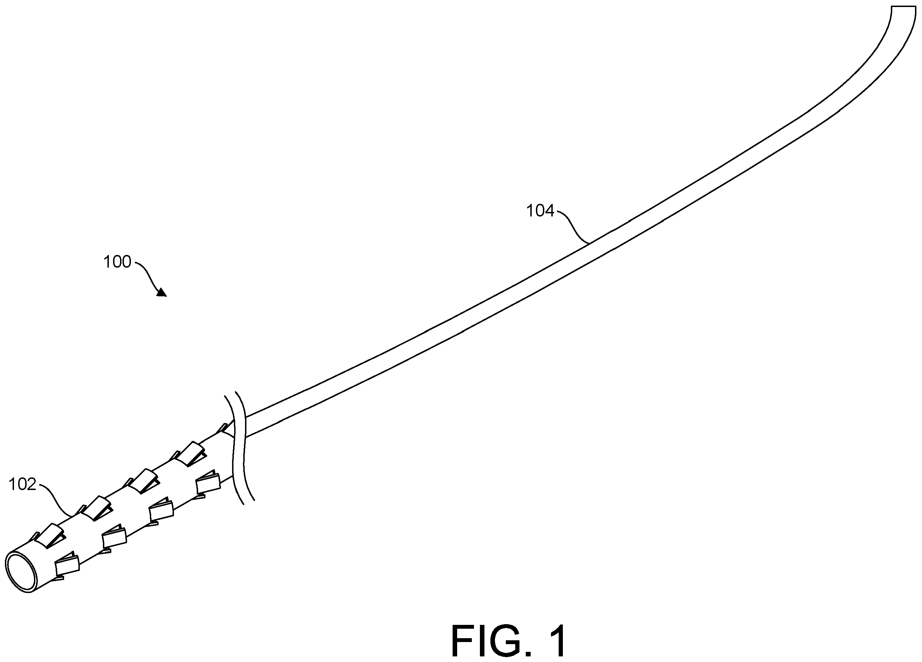

The present disclosure relates generally to lead engagement devices for engaging and facilitating removal of an implanted lead, such as a cardiac implantable electronic device ("CIED") lead, from a patient's body. Referring to FIG. 1, there is shown an exemplary embodiment of the lead engagement devices described herein. The lead engagement device 100 generally includes a distal lead engagement portion 102 and a proximal handle portion 104. FIG. 1 omits a section of the lead engagement device 100, including an interface between the distal lead engagement portion 102 and the proximal handle portion 104, for illustrative purposes. Consequently, FIG. 1 does not illustrate the scaled length of the lead engagement device 100. The lead engagement device 100 may have a length of, for example, at least about 71 cm. However, the lead engagement device 100 may be provided with other lengths depending on the intended application.

With continued reference to FIG. 1, at least a portion of the proximal handle portion 104 remains external to a patient while the distal lead engagement portion 102 enters and engages an implanted lead during a lead removal procedure. As such, traction may be applied to the proximal handle portion 104 to remove the lead from the patient. The proximal handle portion 104 may be a wire, for example, a stainless steel wire, or a continuation of the hypotube without the apertures. The proximal handle portion 104 may include a loop (not shown) at a proximal end portion 106 to facilitate applying traction to the lead engagement device 100.

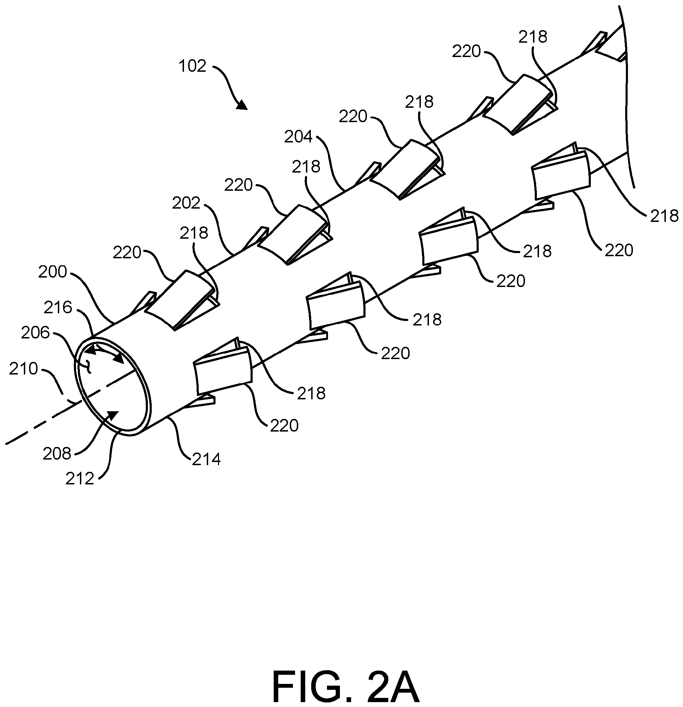

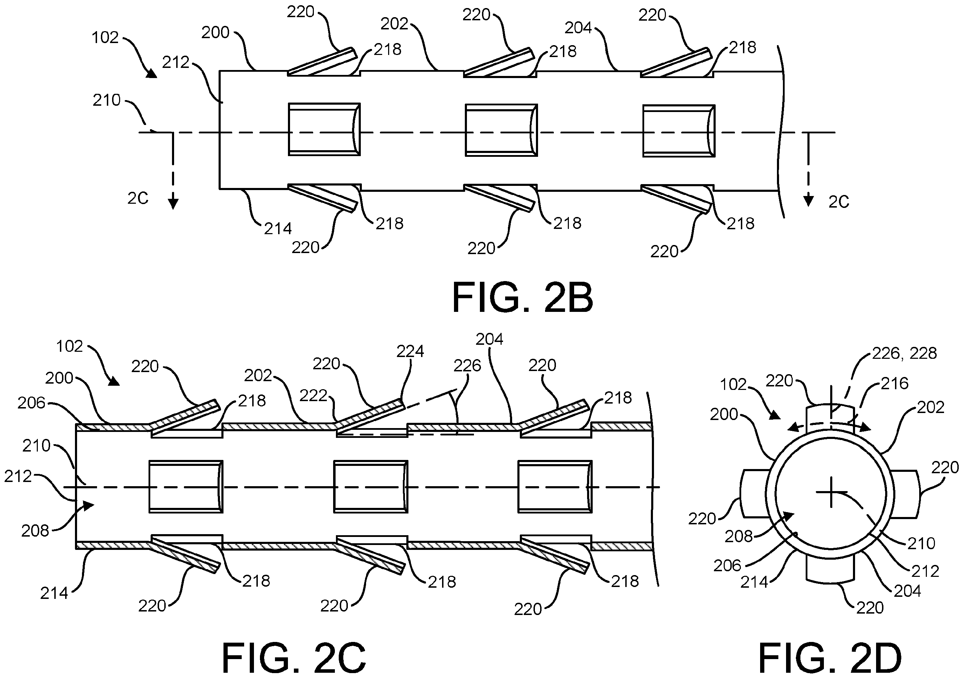

Referring now to FIGS. 2A-2D, the distal lead engagement portion 102 is illustrated. Generally, the distal lead engagement portion 102 is a hypotube 200 comprising one or more of various appropriate materials, such as stainless steel, nitinol, and the like. The hypotube 200 includes a wall 202 that defines an outer surface 204 and an opposite inner surface 206, and the inner surface 206 defines an inner lumen 208 of the hypotube 200. The outer surface 204 may have a diameter that facilitates positioning the distal lead engagement portion 102 within the lumen of lead. The diameter of the outer surface 204 may be, for example, about 0.33 mm to 0.69 mm (0.013 to 0.027 inches). The diameter of the inner surface 206 may be, for example, about 0.25 mm to 0.61 mm (0.010 to 0.024 inches). The inner lumen 208 defines a longitudinal axis 210 that extends from a distal opening 212 at a distal end portion 214 to a proximal opening (not shown) at a proximal end portion (not shown). The inner lumen 208 also defines a circumferential direction 216 that is perpendicular to and extends about the longitudinal axis 210. The wall 202 has a length between the distal opening 212 and the proximal opening of, for example, at least about 71 cm. In some embodiments, the hypotube 200 couples to a coil capped by a half sphere (not shown) near the distal opening 212.

The hypotube 200 further includes a plurality of apertures 218 and a plurality of adjacent lead engagement fingers 220. The apertures 218 and the lead engagement fingers 220 may both be formed, for example, by laser cutting and bending portions of the hypotube 200, as described in further detail below. Generally, the lead engagement fingers 220 extend outwardly from the outer surface 204 of the wall 202 proceeding away from the distal end portion 214 and toward the proximal end portion. Accordingly, the lead engagement fingers 220 engage a lead when the lead engagement portion 102 enters the lumen of the lead and permit unidirectional motion of the lead engagement portion 102 relative to the lead. More specifically, the lead engagement fingers 220 permit relative motion between the lead engagement portion 102 and the lead and slide relative to the lead when applying a first force to the lead engagement portion 102 (for example, applying a pushing force in a generally distal direction). The lead engagement fingers 220 also engage the lead and inhibit relative motion between the lead engagement portion 102 and the lead when applying a second force to the lead engagement portion 102, (for example, applying a pulling or traction force in a generally proximal direction).

In some embodiments and as illustrated most clearly in FIG. 2C, each of the lead engagement fingers 220 has a distal end 222 coupled to the wall 202 and a proximal free end 224 opposite the distal end 222. In some embodiments and as illustrated, the lead engagement fingers 220 are monolithically coupled to and cantilevered from the wall 202 at their distal ends 222. Such a structure permits the lead engagement fingers 220 to (1) deflect radially inwardly and slide across the lead when the lead engagement portion 102 is advanced distally within the lumen of the lead, and (2) engage the lead and inhibit relative motion between the lead engagement portion 102 and the lead when applying traction to the lead engagement device 100.

The lead engagement fingers 220 and the apertures 218 may be provided in a variety of shapes, sizes, and arrangements. The apertures 218 extend through the wall 202 from the inner surface 206 to the outer surface 204. In some embodiments and as illustrated, the plurality of apertures 218 are disposed in planes that are substantially parallel to the longitudinal axis 210 of the hypotube 200. In some embodiments and as illustrated, the lead engagement fingers 220 are normally disposed at acute angles 226 relative to the outer surface 204 ("normally" and variants thereof referring to situations in which external forces are not applied to component). In some embodiments and as illustrated, planes 228 in which the acute angles 226 are disposed (see FIG. 2D) substantially bisect the wall 202 of the hypotube 200, that is, bisect ± 2.54 mm (bisect ± 0.1 inches). In some embodiments and as illustrated, the acute angles 226 are normally in a range of 10 to 30 degrees.

In some embodiments, the lead engagement fingers 220 are generally present along the majority of the length of the hypotube 200. More specifically, the lead engagement fingers 220 may be present over a length in a range of 50 to 75 cm from a most-distal finger to a most-proximal finger. Accordingly, the lead engagement fingers 220 may provide a relatively large area for engaging implanted leads. In some embodiments, multiple lead engagement fingers 220 may be disposed at the same longitudinal position along the wall 202 of the hypotube 200 or grouped at various longitudinal positions. For example and as illustrated, four lead engagement fingers 220 may be disposed at each longitudinal position. Groups of lead engagement fingers 220 may be disposed apart by the same longitudinal distance, as illustrated, or different longitudinal distances. In some embodiments, each lead engagement finger may be disposed at a different longitudinal position than the other lead engagement fingers 220.

The fingers 220 may be provided in a variety of shapes. In some embodiments and as illustrated, each of the fingers 220 has a rectangular shape. The rectangular shape may have a length (between the distal end 222 and the proximal free end 224) of, for example, 0.25 mm to 6.35 mm (0.010 to 0.250 inches). The rectangular shape may have a width (in the circumferential direction 216 of the wall 202) of, for example, 45 degrees. In other embodiments, one or more of the fingers 220 has a different shape, such as a triangular shape, a semi-elliptical shape, a semi-parabolic shape, or the like.

Each aperture 218 may have the same shape as the adjacent finger (although each aperture 218 may be slightly larger than the adjacent finger due to the manner in which the features are formed, as described in further detail below). In some embodiments and as illustrated, each of the apertures 218 has a rectangular shape. In other embodiments, one or more of the apertures 218 has a different shape, such as a triangular shape, a semi-elliptical shape, a semi-parabolic shape, or the like.

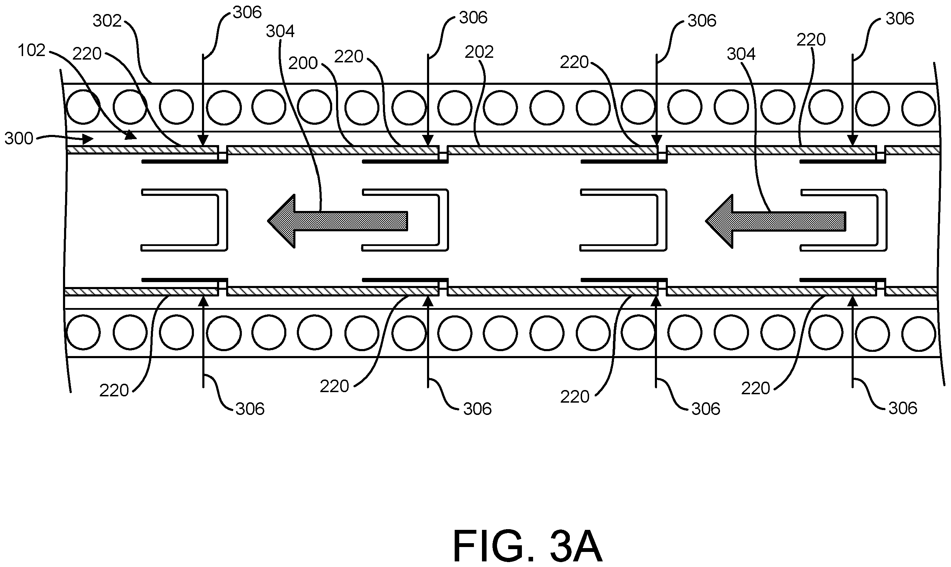

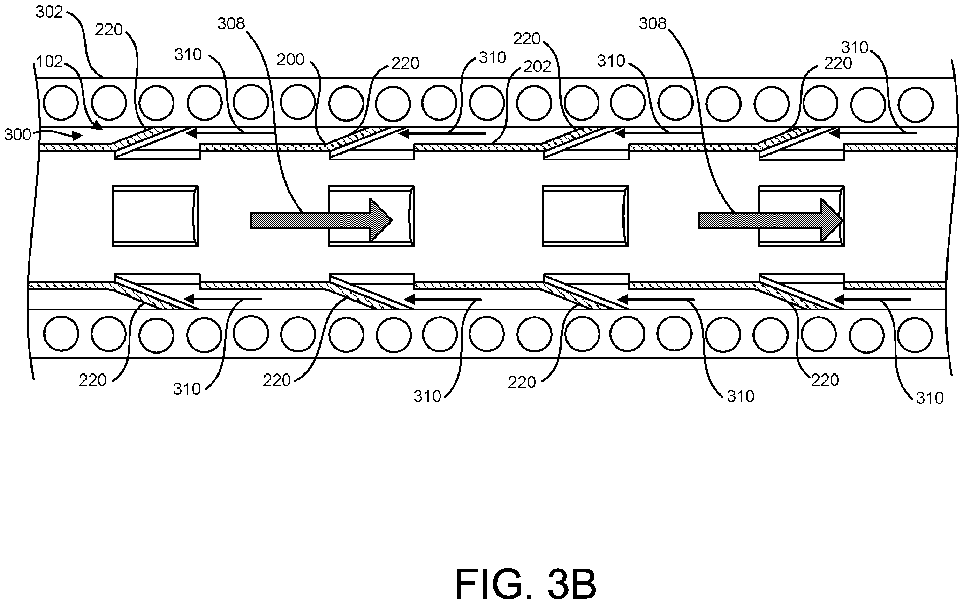

FIGS. 3A and 3B illustrate the lead engagement portion 102 disposed in a lumen 300 of a lead 302. In FIG. 3A, a first or pushing force 304 is applied that urges the lead engagement portion 102 distally relative to the lead 302. In this situation, the lead 302 applies contact forces 306 to the lead engagement fingers 220, and the contact forces 306 cause the fingers 220 to deflect radially inwardly (the degree of deflection is exaggerated in FIG. 3A for illustrative purposes). Accordingly, the fingers 220 slide across the lead 302 and thereby permit the lead engagement portion 102 to move distally in the lumen 300 of the lead 302. In FIG. 3B, a second or pulling force 308 is applied that urges the lead engagement portion 102 proximally relative to the lead 302. However, in this situation the lead 302 applies resistance forces 310 to the fingers 220, and the resistance forces 310 cause the fingers 220 to deflect further radially outwardly. The fingers 220 thereby interfere with the lead 302 and inhibit the lead engagement portion 102 to move proximally in the lumen 300 of the lead 302. As such, the second force 308 acts as a traction force for removing the lead 302 from a patient.

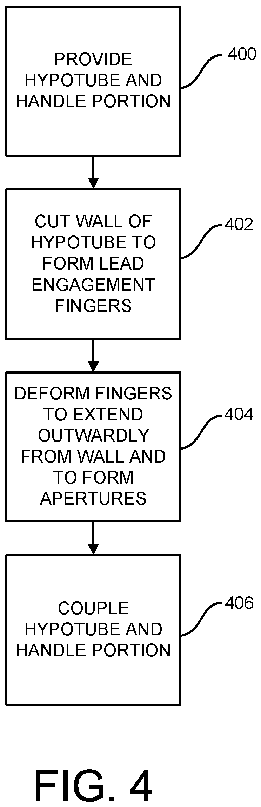

FIG. 4 illustrates an exemplary method for manufacturing a lead engagement device according to the present disclosure. The description of the method refers to the lead engagement device 100 and components described above for illustrative purposes, and the method could be used for manufacturing any of the lead engagement devices according to the present disclosure. The method begins at block 400 by providing the hypotube 200 and the proximal handle portion 104. At block 402, the wall 202 of the hypotube 200 is cut (for example, laser cut) to form the plurality of lead engagement fingers 220. At block 404, the lead engagement fingers 220 are deformed (for example, mechanically bent) such that the fingers 220 normally extend outwardly and proximally from the outer surface 204 of the wall 202 of the hypotube 200 and to form the plurality of apertures 218. At block 406, the hypotube 200 and the proximal portion are coupled to each other (for example, via welding, crimping, or the like).

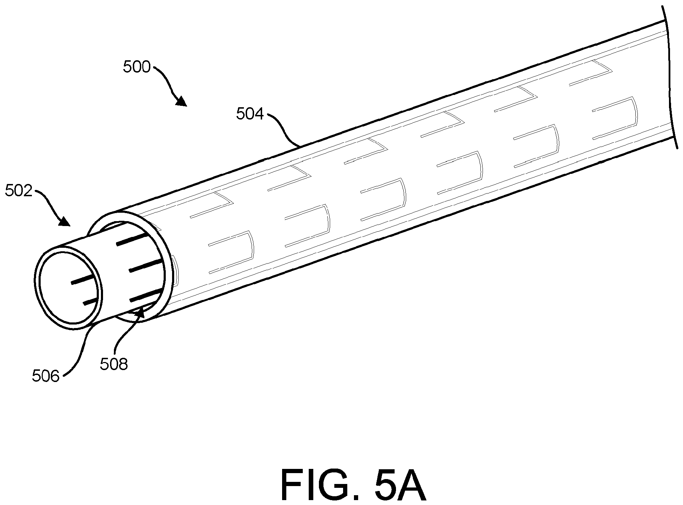

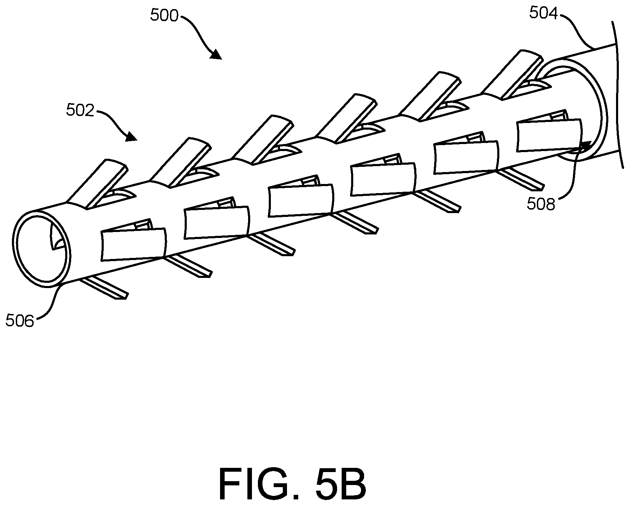

Referring now to FIGS. 5A and 5B, another exemplary embodiment of a lead engagement device 500 according to the present disclosure is illustrated. The lead engagement device 500 generally includes a distal lead engagement portion 502 and a proximal handle portion (not shown), which may be the same or similar to the distal lead engagement portion 102 and a proximal handle portion 104 described above, respectively. The lead engagement device 500 further includes an outer sheath 504 that initially removably receives the hypotube 506 of the distal lead engagement portion 502. FIG. 5A illustrates the lead engagement portion 502 in an initial stowed configuration in which it is disposed within a lumen 508 of the outer sheath 504. FIG. 5B illustrates the lead engagement portion 502 in a deployed configuration in which it is exposed from the outer sheath 504.

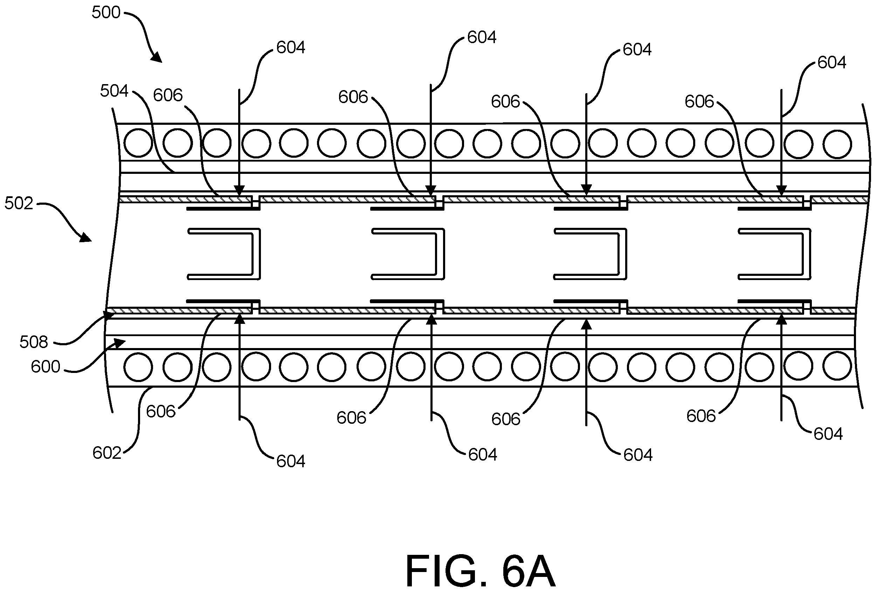

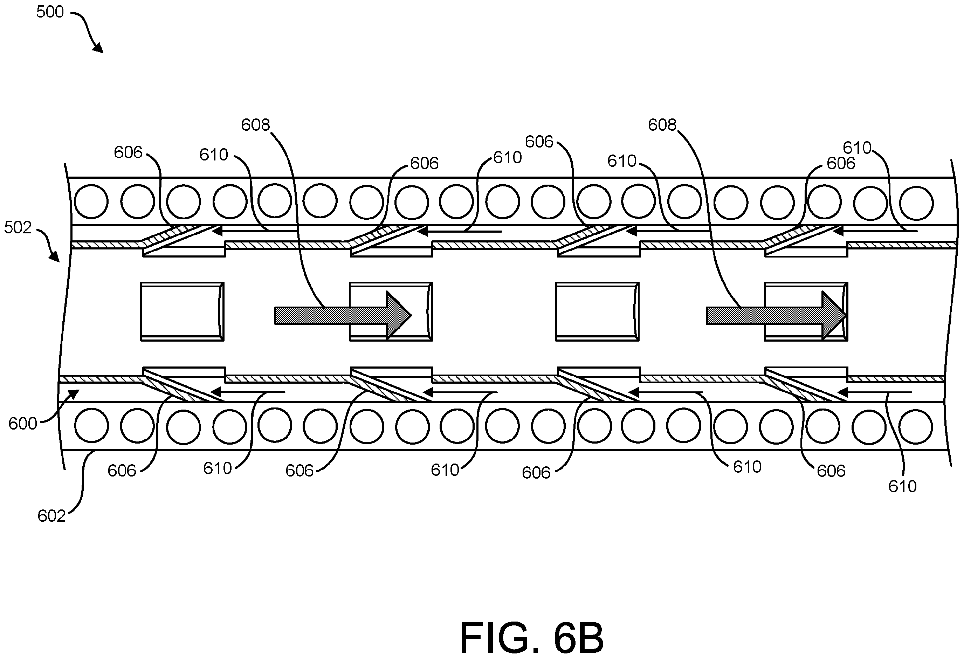

FIGS. 6A and 6B illustrate the lead engagement portion 502 disposed in a lumen 600 of a lead 602. In FIG. 6A, the distal lead engagement portion 502 is in the stowed configuration and received in the lumen 508 of the outer sheath 504. The outer sheath 504 applies contact forces 604 to the lead engagement fingers 606, and the contact forces 604 hold the fingers 606 radially inwardly. Accordingly, the lead engagement fingers 606 do not engage the lead 602 in the stowed configuration, and the distal lead engagement portion 502 and the outer sheath 504 may freely move within the lumen 600 of the lead 602 (both distally and proximally). In FIG. 6B, the distal lead engagement portion 502 is in the deployed configuration (for example, by pulling the outer sheath 504 proximally relative to the distal lead engagement portion 502). In the deployed configuration, a pulling force 608 may be applied that urges the lead engagement portion 502 proximally relative to the lead 602. However, in this situation the lead 602 applies resistance forces 610 to the fingers 606, and the resistance forces 610 cause the fingers 606 to deflect further radially outwardly. The fingers 606 thereby interfere with the lead 602 and inhibit the lead engagement portion 502 to move proximally in the lumen 600 of the lead 602. As such, the pulling force 608 acts as a traction force for removing the lead 602 from a patient.

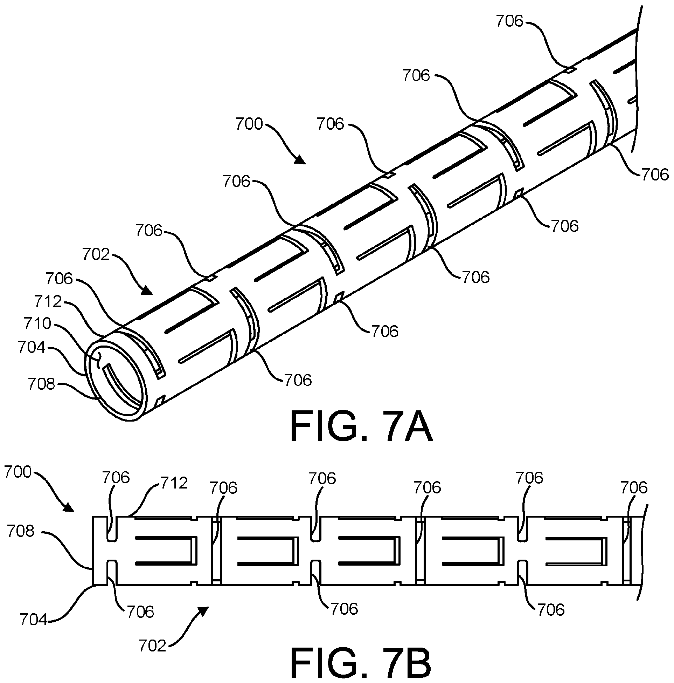

Referring now to FIGS. 7A and 7B, another exemplary embodiment of a lead engagement device 700 according to the present disclosure is illustrated. The lead engagement device 700 generally includes a distal lead engagement portion 702 and a proximal handle portion (not shown), which may be the same or similar to the distal lead engagement portion 102 and a proximal handle portion 104 described above, respectively, except that the hypotube 704 further includes a plurality of tractability-enhancing features that facilitate maneuvering the lead engagement device 700 in tortuous anatomy. In some embodiments and as illustrated, such features may be a plurality of slits 706 that extend through the wall 708 from the inner surface 710 to the outer surface. In some embodiments and as illustrated, the slits 706 may be longitudinally offset from the fingers. In some embodiments and as illustrated, the slits 706 may be grouped at different longitudinal positions. In some embodiments and as illustrated, the slits 706 in each group may be circumferentially offset from the slits 706 in adjacent longitudinal groups.

The foregoing discussion has been presented for purposes of illustration and description. The foregoing is not intended to limit the disclosure to the form or forms disclosed herein. In the foregoing Summary for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Each claim is standing on its own as a separate preferred embodiment of the disclosure.

The invention is defined in the appended claims. Aspects, embodiments and examples disclosed herein which do not fall within the scope of the appended claims do not form part of the invention, and are merely provided for illustrative purposes.

## Claims

1. A lead engagement device (100) configured to be positioned in a lead lumen of a lead, the lead engagement device comprising:
a hypotube (200) comprising:
a wall (202) comprising an inner surface (206) defining an inner lumen (208) and an outer surface (204) opposite the inner surface;
a plurality of apertures (218) extending through the wall (202) from the inner surface to the outer surface; and
a plurality of lead engagement fingers (220) coupled to the wall (202) and extending outwardly and proximally from the outer surface, each of the plurality of lead engagement fingers (220) disposed adjacent to one of the plurality of apertures (218) and comprising a portion of the wall cut to form said one of the apertures, the plurality of lead engagement fingers (220) being configured to permit relative motion between the lead engagement device (100) and the lead when applying a first force to the lead engagement device (100), the plurality of lead engagement fingers (220) being configured to engage the lead and inhibit relative motion between the lead engagement device and the lead when applying a second force to the lead engagement device, the second force being applied in an opposite direction than the first force.

2. The lead engagement device of claim 1, wherein the inner lumen (208) defines a longitudinal axis of the hypotube (200), the plurality of apertures (218) are disposed in planes substantially parallel to the longitudinal axis of the hypotube, and the plurality of lead engagement fingers (220) are normally disposed at acute angles relative to the longitudinal axis.

3. The lead engagement device of claim 2, wherein a plane in which at least one of the acute angles is disposed substantially bisects the wall of the hypotube (200).

4. The lead engagement device of claim 2, wherein at least one of the acute angles is normally in a range of 5 to 45 degrees.

5. The lead engagement device of claim 1, wherein the inner lumen (208) defines a longitudinal axis of the hypotube (200) and circumferential direction extending about the longitudinal axis, wherein the plurality of fingers (220) are curved in the circumferential direction.

6. The lead engagement device of claim 1, wherein at least one of the plurality of lead engagement fingers (220) has a distal end (222) coupled to the wall, a proximal free end (224) opposite the distal end, and length between the distal end and the proximal free end in a range of 0.25 mm to 6.35 mm.

7. The lead engagement device of claim 1, wherein the plurality of lead engagement fingers (220) are cantilevered from the wall and monolithically couple to the wall.

8. The lead engagement device of claim 1, wherein the plurality of lead engagement fingers (220) have a first shape, the plurality of apertures (218) have a second shape, the second shape being the same as the first shape, and preferably the first shape and the second shape are rectangles.

9. The lead engagement device of claim 1, further comprising an outer sheath (504) comprising an outer lumen, the outer lumen removably receiving the hypotube.

10. A method of manufacturing a lead engagement device, the method comprising:
providing a hypotube (200) comprising a wall (202), the wall comprising an inner surface (206) defining an inner lumen (208) and an outer surface (204) opposite the inner surface;
cutting the wall to form a plurality of lead engagement fingers (220) and a plurality of apertures (218) extending through the wall from the inner surface to the outer surface and disposed adjacent to the plurality of lead engagement fingers (220), the lead engagement fingers each comprising a portion of the wall cut to form one of the apertures; and
deforming the plurality of lead engagement fingers such that the plurality of lead engagement fingers (220) normally extend outwardly and proximally from the outer surface.

11. The method of claim 10, wherein cutting the wall (202) comprises laser cutting the wall.

12. The method of claim 10, wherein the inner lumen (208) defines a longitudinal axis of the hypotube (200), wherein deforming the plurality of lead engagement fingers (220) causes the plurality of lead engagement fingers to be normally disposed at acute angles relative to the longitudinal axis and wherein preferably at least one of the acute angles is in a range of 5 to 45 degrees.

13. The method of claim 12, wherein a plane in which at least one of the acute angles is disposed substantially bisects the wall of the hypotube.

14. The method of claim 10, wherein cutting and deforming provides the plurality of lead engagement fingers with a distal end coupled to the wall, a proximal free end opposite the distal end, and length between the distal end and the proximal free end in a range of 0.25 mm to 6.35 mm.

15. The method of claim 10, wherein cutting provides the plurality of lead engagement fingers with rectangular shapes.

## Patentansprüche

1. Leitungseingriffsvorrichtung (100), die konfiguriert ist, um in einem Leitungslumen einer Leitung positioniert zu werden, die Leitungseingriffsvorrichtung umfassend:
ein Hypotubus (200), umfassend:
eine Wand (202), umfassend eine Innenfläche (206), die ein inneres Lumen (208) definiert, und eine Außenfläche (204) gegenüber der Innenfläche;
eine Vielzahl von Öffnungen (218), die sich von der Innenfläche zu der Außenfläche durch die Wand (202) erstrecken; und eine Vielzahl von Leitungseingriffsfingern (220), die mit der Wand (202) gekoppelt sind und sich nach außen und proximal von der Außenfläche erstrecken, wobei jeder der Vielzahl von Leitungseingriffsfingern (220) angrenzend an einer der Vielzahl von Öffnungen (218) angeordnet ist und einen Abschnitt der Wand umfasst, der geschnitten ist, um die eine der Öffnungen zu bilden, wobei die Vielzahl von Leitungseingriffsfingern (220) konfiguriert ist, um eine relative Bewegung zwischen der Leitungseingriffsvorrichtung (100) und der Leitung zuzulassen, wenn eine erste Kraft auf die Leitungseingriffsvorrichtung (100) ausgeübt wird, wobei die Vielzahl von Leitungseingriffsfingern (220) konfiguriert ist, um die Leitung einzugreifen und eine relative Bewegung zwischen der Leitungseingriffsvorrichtung und der Leitung zu verhindern, wenn eine zweite Kraft auf die Leitungseingriffsvorrichtung ausgeübt wird, wobei die zweite Kraft in einer entgegengesetzten Richtung zu der ersten Kraft ausgeübt wird.

2. Leitungseingriffsvorrichtung nach Anspruch 1, wobei das innere Lumen (208) eine Längsachse des Hypotubus (200) definiert, die Vielzahl von Öffnungen (218) in Ebenen angeordnet sind, die im Wesentlichen parallel zu der Längsachse des Hypotubus sind, und die Vielzahl von Leitungseingriffsfingern (220) normalerweise in spitzen Winkeln in Bezug auf die Längsachse angeordnet sind.

3. Leitungseingriffsvorrichtung nach Anspruch 2, wobei eine Ebene, in der mindestens einer der spitzen Winkel angeordnet ist, die Wand des Hypotubus (200) im Wesentlichen halbiert.

4. Leitungseingriffsvorrichtung nach Anspruch 2, wobei mindestens einer der spitzen Winkel normalerweise in einem Bereich von 5 bis 45 Grad ist.

5. Leitungseingriffsvorrichtung nach Anspruch 1, wobei das innere Lumen (208) eine Längsachse des Hypotubus (200) und eine sich um die Längsachse erstreckende Umfangsrichtung definiert, wobei die Vielzahl der Finger (220) in der Umfangsrichtung gekrümmt ist.

6. Leitungseingriffsvorrichtung nach Anspruch 1, wobei mindestens einer der Vielzahl von Leitungseingriffsfingern (220) ein mit der Wand verbundenes distales Ende (222), ein dem distalen Ende gegenüberliegendes proximales freies Ende (224) und eine Länge zwischen dem distalen Ende und dem proximalen freien Ende in einem Bereich von 0,25 mm bis 6,35 mm aufweist.

7. Leitungseingriffsvorrichtung nach Anspruch 1, wobei die Vielzahl von Leitungseingriffsfingern (220) von der Wand freitragend sind und monolithisch mit der Wand gekoppelt sind.

8. Leitungseingriffsvorrichtung nach Anspruch 1, wobei die Vielzahl von Leitungseingriffsfingern (220) eine erste Form aufweisen, die Vielzahl von Öffnungen (218) eine zweite Form aufweisen, wobei die zweite Form gleich wie die erste Form ist, und vorzugsweise die erste Form und die zweite Form Rechtecke sind.

9. Leitungseingriffsvorrichtung nach Anspruch 1, ferner umfassend eine äußere Hülle (504), umfassend ein äußeres Lumen, wobei das äußere Lumen den Hypotubus abnehmbar aufnimmt.

10. Verfahren zum Herstellen einer Leitungseingriffsvorrichtung, das Verfahren umfassend:
Bereitstellen eines Hypotubus (200), umfassend eine Wand (202), die Wand umfassend eine Innenfläche (206), die ein inneres Lumen (208) definiert, und eine Außenfläche (204) gegenüber der Innenfläche;
Schneiden der Wand, um eine Vielzahl von Leitungseingriffsfingern (220) und eine Vielzahl von Öffnungen (218) zu bilden, die sich durch die Wand von der inneren Oberfläche zu der Außenfläche erstrecken und neben der Vielzahl von Leitungseingriffsfingern (220) angeordnet sind, die Leitungseingriffsfinger jeweils umfassend einen Abschnitt der Wand, der geschnitten ist, um eine der Öffnungen zu bilden; und
Verformen der Vielzahl von Leitungseingriffsfingern, sodass sich die Vielzahl von Leitungseingriffsfingern (220) normalerweise nach außen und proximal von der Außenfläche erstrecken.

11. Verfahren nach Anspruch 10, wobei ein Schneiden der Wand (202) ein Laserschneiden der Wand umfasst.

12. Verfahren nach Anspruch 10, wobei das innere Lumen (208) eine Längsachse des Hypotubus (200) definiert, wobei ein Verformen der Vielzahl von Leitungseingriffsfingern (220) bewirkt, dass die Vielzahl von Leitungseingriffsfingern normalerweise in spitzen Winkeln in Bezug auf die Längsachse angeordnet ist, und wobei vorzugsweise mindestens einer der spitzen Winkel in einem Bereich von 5 bis 45 Grad ist.

13. Verfahren nach Anspruch 12, wobei eine Ebene, in der mindestens einer der spitzen Winkel angeordnet ist, die Wand des Hypotubus im Wesentlichen halbiert.

14. Verfahren nach Anspruch 10, wobei ein Schneiden und Verformen die Vielzahl von Leitungseingriffsfingern mit einem distalen Ende, das mit der Wand verbunden ist, einem proximalen freien Ende gegenüber dem distalen Ende und einer Länge zwischen dem distalen Ende und dem proximalen freien Ende in einem Bereich von 0,25 mm bis 6,35 mm versieht.

15. Verfahren nach Anspruch 10, wobei ein Schneiden die Vielzahl der Leitungseingriffsfinger mit rechteckigen Formen versieht.

## Revendications

1. Dispositif d'engagement de conducteur (100) configuré pour être positionné dans une lumière de conducteur d'un conducteur, le dispositif d'engagement de conducteur comprenant:
un hypotube (200) comprenant:
une paroi (202) comprenant une surface interne (206) définissant une lumière interne (208) et une surface externe (204) opposée à la surface interne;
une pluralité d'ouvertures (218) s'étendant à travers la paroi (202) de la surface intérieure à la surface extérieure; et
une pluralité de doigts d'engagement de conducteur (220) couplés à la paroi (202) et s'étendant vers l'extérieur et de manière proximale à partir de la surface externe, chacun de la pluralité de doigts d'engagement de conducteur (220) étant disposé adjacent à l'une de la pluralité d'ouvertures (218) et comprenant une partie de la paroi découpée pour former ladite une des ouvertures, la pluralité de doigts d'engagement de conducteur (220) étant configurés pour permettre un mouvement relatif entre le dispositif d'engagement de conducteur (100) et le conducteur lors de l'application d'une première force à l'engagement de conducteur dispositif (100), la pluralité de doigts d'engagement de conducteur (220) étant configurée pour engager le conducteur et inhiber le mouvement relatif entre le dispositif d'engagement de conducteur et le conducteur lors de l'application d'une seconde force au dispositif d'engagement de conducteur, la seconde force étant appliquée dans un sens opposé à la première force.

2. Dispositif d'engagement de conducteur selon la revendication 1, dans lequel la lumière intérieure (208) définit un axe longitudinal de l'hypotube (200), la pluralité d'ouvertures (218) est disposée dans des plans sensiblement parallèles à l'axe longitudinal de l'hypotube, et la pluralité des doigts d'engagement de conducteur (220) est normalement disposée à des angles aigus par rapport à l'axe longitudinal.

3. Dispositif d'engagement de conducteur selon la revendication 2, dans lequel un plan dans lequel au moins l'un des angles aigus est disposé coupe sensiblement en deux la paroi de l'hypotube (200).

4. Dispositif d'engagement de conducteur selon la revendication 2, dans lequel au moins l'un des angles aigus est normalement dans une plage de 5 à 45 degrés.

5. Dispositif d'engagement de conducteur selon la revendication 1, dans lequel la lumière intérieure (208) définit un axe longitudinal de l'hypotube (200) et une direction circonférentielle s'étendant autour de l'axe longitudinal, dans lequel la pluralité de doigts (220) est incurvée dans la direction circonférentielle.

6. Dispositif d'engagement de conducteur selon la revendication 1, dans lequel au moins l'un de la pluralité de doigts d'engagement de conducteur (220) a une extrémité distale (222) couplée à la paroi, une extrémité libre proximale (224) opposée à l'extrémité distale, et une longueur entre l'extrémité distale et l'extrémité libre proximale dans une plage de 0,25 mm à 6,35 mm.

7. Dispositif d'engagement de conducteur selon la revendication 1, dans lequel la pluralité de doigts d'engagement de conducteur (220) est en porte-à-faux depuis la paroi et couplée de manière monolithique à la paroi.

8. Dispositif d'engagement de conducteur selon la revendication 1, dans lequel la pluralité de doigts d'engagement de conducteur (220) a une première forme, la pluralité d'ouvertures (218) a une seconde forme, la seconde forme étant la même que la première forme, et de préférence la première forme et la deuxième forme sont des rectangles.

9. Dispositif d'engagement de conducteur selon la revendication 1, comprenant en outre une gaine externe (504) comprenant une lumière externe, la lumière externe recevant de manière amovible l'hypotube.

10. Procédé de fabrication d'un dispositif d'engagement de conducteur, le procédé consistant à:
fournir un hypotube (200) comprenant une paroi (202), la paroi comprenant une surface interne (206) définissant une lumière interne (208) et une surface externe (204) opposée à la surface interne;
couper la paroi pour former une pluralité de doigts d'engagement de conducteur (220) et une pluralité d'ouvertures (218) s'étendant à travers la paroi de la surface interne à la surface externe et disposée adjacente à la pluralité de doigts d'engagement de conducteur (220), le conducteur des doigts d'engagement comprenant chacun une partie de la paroi découpée pour former l'une des ouvertures; et
déformer la pluralité de doigts d'engagement de conducteur de sorte que la pluralité de doigts d'engagement de conducteur (220) s'étend normalement vers l'extérieur et de manière proximale à partir de la surface externe.

11. Procédé selon la revendication 10, dans lequel la découpe de la paroi (202) comprend la découpe au laser de la paroi.

12. Procédé selon la revendication 10, dans lequel la lumière interne (208) définit un axe longitudinal de l'hypotube (200), dans lequel la déformation de la pluralité de doigts d'engagement de conducteur (220) amène la pluralité de doigts d'engagement de conducteur à être normalement disposés à des angles aigus par rapport à l'axe longitudinal et dans lequel, de préférence, au moins un des angles aigus est dans une plage de 5 à 45 degrés.

13. Procédé selon la revendication 12, dans lequel un plan dans lequel au moins l'un des angles aigus coupe sensiblement en deux la paroi de l'hypotube.

14. Procédé selon la revendication 10, dans lequel la coupe et la déformation fournissent la pluralité de doigts d'engagement de conducteur avec une extrémité distale couplée à la paroi, une extrémité libre proximale opposée à l'extrémité distale, et une longueur entre l'extrémité distale et l'extrémité libre proximale dans une plage de 0,25 mm à 6,35 mm.

15. Procédé selon la revendication 10, dans lequel la découpe donne à la pluralité de doigts d'engagement de conducteur des formes rectangulaires.
